# EUROPEAN PATENT APPLICATION

(11) **EP 0 973 037 A1**
(43) Date of publication of application: **19.01.2000**
(21) Application number: 99202284.8
(22) Date of filing: 12.07.1999
(51) Int. Cl.: G01N 33/68, G01N 33/53, G01N 33/50

(54) **Method for monitoring the extra-pyramidal effects of antipsychotic agents**

(30) Priority: 14.07.1998 IL 12534698
(71) Applicant: BEN-GURION UNIVERSITY OF THE NEGEV, Beer-Sheva (IL)
(72) Inventor: Schreiber-Avissar, Sofia, 84 965 Omer (IL)
(74) Representative: Long, Giorgio

(57) **Abstract**

A method for monitoring the course and extent of the extrapyramidal side-effects of anti-psychotic agents and/or predicting their occurrence, in a patient having a psychotic disorder, undergoing or commencing treatment with an anti-psychotic agent, comprising the steps of:
a. determining the G-protein levels (G-LEVs) of the subject;
b. determining whether said G-LEVs are lower than those of healthy subjects; and
c. if said G-LEVs are lower than those of healthy subjects, diagnosing the subject as suffering from, or likely to suffer from extrapyramidal symptoms.

## Description

### Field of the Invention

The present invention is concerned with the use of measurements of G-protein activity and/or concentration in order to monitor the occurrence and extent of extra-pyramidal side-effects in patients treated with antipsychotic drugs.

### Background of the Invention

Currently used diagnostic methods for psychiatric disorders tend to be phenomenological and empirical in type. This is in sharp contrast to many other branches of medicine, where advances in the understanding of molecular mechanisms of disease have led to more rational approaches to both its classification and control.

In recent years, however, research addressing biochemical mechanisms underlying the pathogenesis and treatment of neuropsychiatric disorders has focused on alterations in the post-receptor information transduction elements. The family of heterotrimeric G proteins is a crucial point of convergence in the transmission of signals from a variety of hormone and neurotransmitter membrane receptors to a series of downstream cellular events: intracellular second messenger effector enzymes and ionic channels. It has been shown that receptor binding of agonists leads to displacement of GDP from the a subunit of the G-protein by GTP. In the case of the stimulatory G proteins (Gs), this change in nucleotide binding causes activation of the G-protein, wherein there is dissocation of the β and γ subunits. Gsα moves towards adenylate cyclase, leading to its activation and the subsequent catalytic production of the 2^{nd} messenger cAMP from ATP.

The increasing interest in the clinical perspective of altered G protein function has yielded important findings concerning the involvement of G proteins in the pathophysiology of mood disorders and in the biochemical mechanisms underlying the treatment of these disorders. The function of receptor-coupled G proteins was found to be altered by lithium, and other antibipolar treatments (Avissar S., Schreiber G., *Pharmacopsychiatry* 1992;25:44-50). Increased activity of Gₛ and Gᵢ proteins, and elevated levels of Gsα and Giα subunit proteins were detected in mononuclear leukocytes (MNL) of patients with mania (Avissar S. et al., *J Affect Disord.* 1997;43:85-93.). In contrast to the findings in manic patients, reduced functional measures of Gₛ and Gᵢ proteins have been reported in MNL of patients with major depressive disorder (*ibid.*). Although conflicting results were obtained concerning G proteins immunoreactive levels in MNL of patients with major depression, a larger scale study has detected reduced levels of Gsα and Giα proteins in MNL of depressed patients which were in correlation with the severity of depression and with the reductions in the functional measures of these proteins (Avissar S, et al. *Am J Psychiat* 1997; 154:211-217.). These novel and unexpected alterations in G-protein activity and concentration in psychiatric diseases form the basis of the differential diagnostic methods disclosed in a co-owned patent application, WO 97/20211.

Quantitative and functional measures of G proteins have also been undertaken in mononuclear leukocytes of patients with Parkinson's disease and in patients with schizophrenia. Both β-adrenergic and dopamine receptor-coupled Gs protein function, measured through isoproterenol and dopamine-enhanced guanine nucleotide binding capacity, and Gas protein immunoreactivity, were found to be significantly reduced in MNL of patients with Parkinson's disease in comparison with healthy volunteer subjects of similar age (Avissar, S. et al., *Movement Disorders* 12:167-174, 1997).

In contrast, it has now been surprisingly found that MNL of patients with predominantly positively symptomatic schizophrenia present significantly increased dopamine-enhanced guanine nucleotide binding capacity to Gs protein in comparison with healthy subjects. Values for β-adrenergic and muscarinic receptor-coupled G protein activity, as well as for Gsα, Giα and Gβ immunoreactivities in schizophrenic patients are similar to those in healthy subjects.

Dopamine antagonists are used as antipsychotic medications. "Classical" antipsychotics (mostly D2 and D1 receptor antagonists), especially the high potency antipsychotics, such as haloperidol, very commonly cause extrapyramidal side effects with parkinsonism as one of the most prominent. For some of these antipsychotic agents, for example risperidone, the aforementioned side effects are dose-related. In addition, newly developed antipsychotics, the DS (dopamine-serotonin) antagonists, of which clozapine is the prototype, are known to be without extrapyramidal side effects.

### SUMMARY OF THE INVENTION

It has now been surprisingly found, and this is an object of the invention, that it is possible to use measurement of G-protein levels to provide an objective and quantitative assessment of the extra-pyramidal side effects of the anti-psychotic drugs.

The invention is primarily directed to a method for monitoring the course and extent of the extrapyramidal side-effects of anti-psychotic agents and/or predicting their occurrence, in a patient having a psychotic disorder, undergoing or commencing treatment with an anti-psychotic agent, comprising the steps of:
a. determining the G-protein levels (G-LEVs) of the subject;
b. determining whether said G-LEVs are lower than those of healthy subjects; and
c. if said G-LEVs are lower than those of healthy subjects, diagnosing the subject as suffering from, or likely to suffer from extrapyramidal symptoms.

One aspect of the invention is directed to the measurement of G protein levels by the assessment of agonist-stimulated G-protein function.

In a preferred embodiment, the agonist used in stimulating G-protein function is dopamine.

In yet another preferred embodiment, the agonist used in stimulating G-protein function is isoproterenol.

The invention also provides for the determination of G-LEVS by the measurement of immunoreactive levels of the G protein subunit Gsα.

In another aspect, the invention is directed to a method for predicting the appearance of extrapyramidal side-effects, wherein the patient is within the first four weeks of treatment with the anti-psychotic drug.

In a further aspect, the invention is directed to the use of the aforementioned methods for determining G-LEVs in the quantitation of extrapyramidal side-effects of anti-psychotic drugs in a patient who has been treated with the anti-psychotic agent for a period of more than four weeks.

The invention also encompasses a method for measuring G-LEVs, wherein the patient is being treated with an anti-psychotic agent that causes dose-dependent extrapyramidal side-effects, and whereby the results of the method are used to determine the drug dosage that will give the most favorable therapeutic index. In one preferred embodiment, this method is applied to a patient being treated with the anti-psychotic drug risperidone.

In another aspect, the invention is directed to a method, wherein the measurement of G-LEVs is applied to a patient being treated with a candidate anti-psychotic drug, the results thereby obtained being used in the selection and development of new anti-psychotic agents having low and/or dose-dependent extra-pyramidal side effects.

All of the above methods of the invention may be used in relation to any psychotic disorder, but most preferably when the psychotic disorder is schizophrenia.

The invention is also directed to a kit for monitoring extra-pyramidal side-effects by the use of G-protein activity and/or concentration measurements, according to any of the aforementioned methods, the said kit comprising:
i. labelled guanine nucleotide;
ii. agonists: isoproterenol, carbamylcholine and dopamine;
iii. antibodies directing specifically against the G protein subunit Gsα;
iv. reference values for interpretation of results.

In another embodiment, the above-described kit further comprises separation media for the purification of MNL.

The invention further provides for any of the kits previously described, but further comprising:
i. homogenization buffer;
ii. guanine nucleotide binding assay buffer

All the above and other characteristics and advantages of the invention will be further understood from the following illustrative and non-limitative examples of preferred embodiments thereof.

### Brief Description of the Drawings

The present invention will be more clearly understood from the detailed description of the preferred embodiments and from the attached drawings in which:
Fig 1 is a bar graph summarizing the results of measurements of dopamine-enhanced Gpp binding. The results are expressed as the percentage increase in Gpp binding capacity that occurs on addition of dopamine (50 µM) to the reaction mixture.
Fig. 2 is a scatter plot indicating the correlation between the results of agonist-enhanced Gpp binding assays and a clinical assessment of the intensity of extrapyrimidal side effects of anti-psychotic agents, as measured on the Angus rating scale.
Fig. 3 is a graph showing the effects of haloperidol and clozapine on the immunoreactivities of Gs and Gi alpha subunit proteins in MNL of patients with schizophrenia.

### Detailed Description of Preferred Embodiments

For purposes of clarity and as an aid in the understanding of the invention, as disclosed and claimed herein, the following terms and abbreviations are defined below:
- **Psychotic disorder** -: a mental and behavioral disorder causing gross distortion or disorganization of a person's mental capacity, affective response, and capacity to recognize reality, communicate and relate to others, to the degree of causing incapacity to cope with the ordinary demands of everyday life.
- **Mononuclear leukocytes (MNLs)** -: herein used to refer to the isolated fraction of peripheral blood cells containing lymphocytes and monocytes.
- **G-proteins** -: GTP-binding regulatory proteins involved in cell signaling processes. There exist a number of sub-types including: stimulatory (Gs), inhibitory (Gi), and phosphoinositide-specific phospholipase C-related G proteins (Gp). Most G proteins are heterotrimers, comprising an α chain loosely bound to a βγ dimer.

### Example 1

### Measurement of agonist-enhanced G protein function

### 1. Subjects

Isoproterenol, dopamine and carbamylcholine enhanced [³H]Gpp(NH)p binding capacities to G proteins in mononuclear leukocytes were compared among the following groups:
A: healthy volunteers;
B: untreated patients with predominantly positively symptomatic schizophrenia;
C: patients with schizophrenia under haloperidol treatment;
D: patients with schizophrenia under clozapine treatment;
E: untreated patients with Parkinson's disease.

All patients were diagnosed according to DSM-IV criteria by at least two senior psychiatrists, using structured clinical interviews: SCID (DSM-IV). Inclusion criteria were normal results of physical examination, electrocardiogram, and laboratory tests for renal, hepatic, hematologic, and thyroid function. No treatment was given during one month prior to referral. The group of schizophrenic patients with positive symptoms consisted of 13 male and 10 female hospitalized subjects, average age 31.4 (19-59) years, diagnosed as suffering from schizophrenia of paranoid or disorganized types with a course classified as acute, subchronic with acute exacerbation, or chronic with acute exacerbation. This group of patients presented predominantly positive symptomatology, assessed by the Positive and Negative Syndrome Scale, PANSS (Total PANSS Score=92.8±5.5; Positive Scale=26.7±1.5). Eight of the patients, never before treated by dopamine antagonists, were examined during theft first psychotic episode and the diagnosis of schizophrenia was reached after at least one year of clinical follow-up. The group of patients under haloperidol or clozapine treatment was evaluated to have less symptomology: total PANSS score = 45.0 ± 5.3; positive scale = 13.4 ± 1.9. The healthy volunteer group consisted of 17 men and 15 women, average age 33.2 (20-62) years, from the medical staff and staff's families of the Beer-Sheva Mental Health Center.

### 2. Methods

Mononuclear leukocytes were isolated from 60 ml heparinized fresh blood of adult donors, using a Ficoll-Paque gradient. Cells were homogenized in 25 mM Tris-HCl, pH 7.4, and 1 mM dithiotreitol (DTT), and membranes were collected by further centrifugation at 18,000 g for 20 min. Membranes were either freshly used for the functional binding measures or suspended in homogenization buffer containing 1mM ATP, 1mM EGTA, 2mM Mg²⁺ and 30% sucrose w/v and stored at -70°. Aliquots were taken for protein concentration determination using the Bradford assay.

Guanine nucleotide binding was estimated as follows: binding was carried out for 10 min. at 25°C in a final volume of 100 µl The reaction buffer consisted of 25 mM Tris-HCl, pH 7.4, 1 mM ATP, 1 mM Mg²⁺, 1 mM EGTA, and 1 mM DTT, and 0.5 - 5 µM ³H-Gpp(NH)p. ³H-Gpp(NH)p binding was carried out in triplicate. Nonspecific binding was measured in samples containing 100 µM unlabeled GTPγS for determination of nonspecific. Specific binding was calculated by subtracting the nonspecific from the total binding. Reactions were started by adding 50 mg of membrane protein and terminated with 5 ml of ice-cold buffer containing 10 mM Tris-HCl, pH 7.4, and 100 mM NaCl, and filtration through GF/C Whatman filters. Filters were subsequently washed twice with 3 ml of cold buffer and taken for scintillation counting. Agonist effects on 3H-Gpp(NTH)p binding were assessed by adding isoproterenol (25 µM) or carbamylcholine (50 µM) or dopamine (50 µM) to the reaction mixture. These represent the minimal concentrations that result in maximal effect of the agonists.

Statistical significance of differences between subject groups with respect to agonist-enhanced guanine nucleotide binding was tested by use of Dunn's non-parametric test for multiple comparisons (Glantz, S.A. Biostatstics, 3^{rd} ed. New York, 1992).

### 3. Results

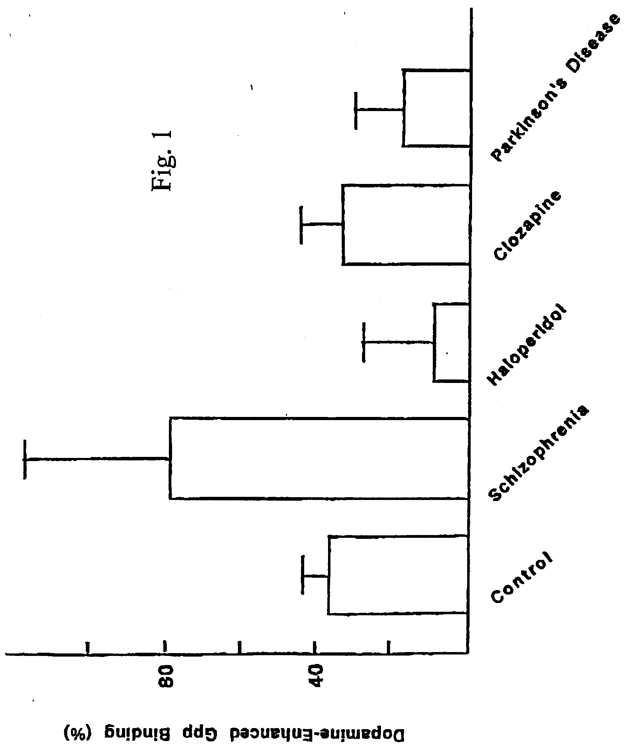

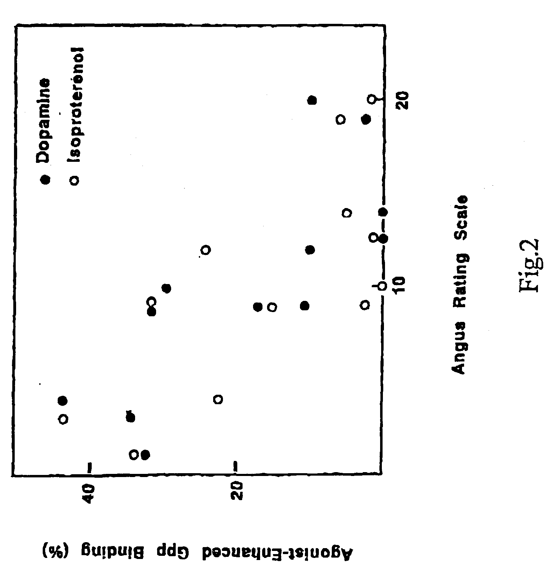

While the untreated schizophrenic show elevated dopamine enhanced Gpp(NH)p binding to Gs protein, untreated patients with Parkinson's disease demonstrate a decrease in both dopamine-enhanced, and isoproterenol-enhanced guanine nucleotide binding to Gs protein (Figure 1, Table I). The prototypical dopamine antagonist antipsychotic agent, haloperidol caused a significant decrease in agonist-enhanced Gpp(NH)p binding capacity (for isoproterenol: Q'=2.29, v=3, p<0.05; for dopamine: Q'= 2.42, v=3, p<0.05; for carbamylcholine: Q'=4.03, v=3, p<0.01) (Figure 1, Table I). The extent of reduction in β-adrenergic and dopaminergic receptors-coupled Gs protein function induced by haloperidol was found to be correlated with the intensity of extrapyramidal side effects clinically induced in the patients as measured by the Angus Scale (Allan E.R, Psychopharmacol. Bull., 34: 71-74, 1998) (Figure 2). Dopamine-enhanced Gpp(NH)p binding capacity to Gs in haloperidol-treated patients was found to be significantly negatively correlated with the Angus Scale ratings: Spearman's correlation coefficient, r=-0.664, p<0.02. For both β-adrenergic and dopaminergic agonists effects: r=0.592, p<0.005. In contrast, the prototypical dopamine-serotonin antagonist, clozapine, had no effects on receptor-coupled Gs and Gi proteins function (for isoproterenol: Q'=0.39, v=3, N.S.; for dopamine: Q'=1.85, v=3, N.S; for carbamylcholine: Q'=1.91, v=3, N.S.).

**Table I**

| | **Receptor-coupled G protein function** | | |
|---|---|---|---|
| | **β-Gs** | **M-Gi** | **D-Gs** |
| **Healthy volunteers** | 26.7(7.0) | 30.4(7.7) | 26.8(9.8) |
| **Schizophrenia** | 29.1(7.5) | 28.0(8.1) | 76.5(38.5) |
| **Schizophrenia treated with haloperidol** | 4.8(18.3) | 2.7(15.9) | 9.5(19.0) |
| **Schizophrenia treated with clozapine** | 25(15.7) | 9.2(14.3) | 36.8(12.1) |
| **Parkinson's disease** | 17.1(13.8) | 21.7(13.8) | 18.1(12.8) |

### Example 2

### Measurement of G protein subunit levels

### 1. Subjects

G-protein subunit immunoreactivities were measured in mononuclear leukocytes obtained using the same method and from the same subject groups described in Example 1.

### 2. Methods

On the day of the assay, MNL membranes (purified as described in Example 1) were thawed, and 10 µg aliquots were separated on SDS polyacrylamide (10%) gel electrophoresis. The separated proteins were transferred by Western blotting to nitrocellulose paper. Blots were blocked with 5% BSA for 1 hr in TBS containing 0.1% Tween -20 (TTBS) and incubated overnight with each of the following polyclonal antisera (NEN-DuPont) at the titres indicated: anti-Gsα 1:2,500, anti-Gα_{i1,2} 1:5,000, and anti-G_{β} 1:1,000. The blots were subsequently incubated with goat anti-rabbit IgG labeled with horseradish peroxidase. Immunoreactivity was detected with the Enhanced Chemiluminescence Western Blot Detection System (Amersham) followed by exposure to Kodak X-Omat film. The range of linearity of the assay with respect to protein concentration was found to be between 2.5 - 15 µg membrane protein. The immunoreactive band intensities were measured with an image analysis system. 10 µg of rat cortical membranes were run on each blot as an internal standard.

Statistical analysis was performed as described in Example 1.

### 3. Results

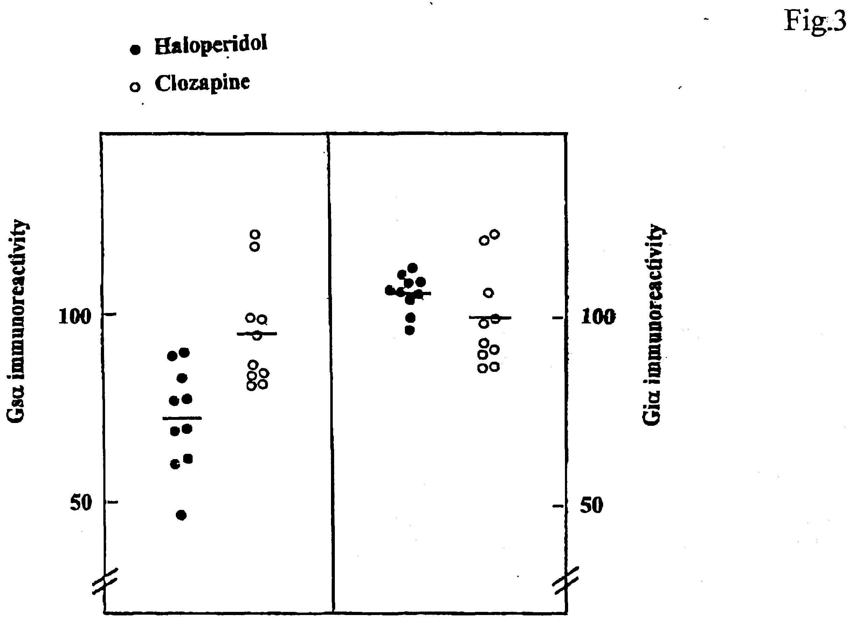

Immunoblot analyses using polyclonal antibodies against Gsα and Giα proteins showed that the immunoreactive levels of these proteins in MNL of untreated patients with schizophrenia of predominantly positive symptoms were similar to (G protein levels in MNL of healthy volunteers (Table II). MNL of patients with schizophrenia under treatment with haloperidol showed significantly decreased Gsα inmunoreactivity (Q'=4.44, v=3, p<0.01, Dunn's Test), while Giα levels were found to be similar to those obtained in healthy subjects (Q'=1.11, v=3, N.S., Dunn's Test) (Figure 3, Table II). This pattern of G protein subunit levels in patients with schizophrenia under haloperidol treatment is similar to the one obtained in MNL of patients with Parkinson's disease (Table II). Patients with schizophrenia under clozapine treatments showed Gsα and Giα protein levels similar to healthy subjects (Q'=1.52, v=3, N.S. and Q'=0.34, v=3, N.S., respectively, Dunn's Test) (Figure 3, Table II).

**Table II**

| | **G protein immunoreactivities** | |
|---|---|---|
| | **Gsα** | **Giα** |
| **Healthy volunteers** | 100.0(8.1) | 100.0(7.5) |
| **Schizophrenia** | 98.3(13.7) | 95.4(11.2) |
| **Schizophrenia treated with haloperidol** | 72.7(14.0) | 105.8(5.7) |
| **Schizophrenia treated with clozapine** | 94.6(16.3) | 98.8(13.7) |
| **Parkinson's disease** | 72.6(21.8) | 100.3(19.3) |

## Claims

1. A method for monitoring the course and extent of the extrapyramidal side-effects of anti-psychotic agents and/or predicting their occurrence, in a patient having a psychotic disorder, undergoing or commencing treatment with an anti-psychotic agent, comprising the steps of:
a. determining the G-protein levels (G-LEVs) of the subject;
b. determining whether said G-LEVs are lower than those of healthy subjects; and
c. if said G-LEVs are lower than those of healthy subjects, diagnosing the subject as suffering from, or likely to suffer from extrapyramidal symptoms.

2. A method according to claim 1, wherein the G-LEVs are measured as agonist-stimulated G-protein function.

3. A method according to claim 2, wherein the agonist is dopamine.

4. A method according to claim 2, wherein the agonist is isoproterenol.

5. A method according to claim 1, wherein the G-LEVs are determined by measurement of immunoreactive levels of the G protein subunit Gsα.

6. A method according to any of claims 1 to 5, wherein the patient is within the first four weeks of treatment with the anti-psychotic agent, and whereby a prediction of the appearance of extrapyramidal side-effects is made.

7. A method according to any of claims 1 to 5, wherein the patient has been treated with the anti-psychotic agent for a period of more than four weeks, and whereby the results of the method are used to quantitate the extrapyramidal side-effects.

8. A method according to any of claims 1 to 5, wherein the patient is being treated with an anti-psychotic agent that causes dose-dependent extrapyrimidal side-effects, and whereby the results of the method are used to determine the drug dosage that will give the most favorable therapeutic index.

9. A method according to claim 8, wherein the anti-psychotic drug is risperidone.

10. A method according to any of claims 1 to 5, wherein the patient is being treated with a candidate anti-psychotic drug, comprising using the results thereby obtained in the selection and development of new anti-psychotic agents having low and/or dose-dependent extra-pyramidal side effects.

11. A method according to any of the above claims, wherein the psychotic disorder is schizophrenia.

12. A method for treating a patient suffering from a psychotic disorder, with a drug inducing extra-pyramidal side-effects, comprising testing the G-LEV of the patient being treated, and reducing the dosage of the drug or shortening the treatment period, if the measured G-LEV indicates the expected appearance of extra-pyramidal side-effects.

13. A kit for monitoring extra-pyramidal side-effects by the use of G-protein activity and/or concentration measurements, according to the methods of any of claims 1 to 12, comprising:
i. labelled guanine nucleotide;
ii. agonists: isoproterenol, carbamylcholine, dopamine;
iii. antibodies directing specifically against the G protein subunit Gsα;
iv. reference values for interpretation of results.

14. A kit according to claim 13, further comprising separation media for the purification of MNL.

15. A kit according to claims 13 or 14, further comprising:
i. homogenization buffer;
ii. guanine nucleotide binding assay buffer.
